# EUROPEAN PATENT APPLICATION

(11) **EP 0 709 100 A1**
(43) Date of publication of application: **01.05.1996**
(21) Application number: 95114718.0
(22) Date of filing: 19.09.1995
(51) Int. Cl.: A61K 49/00

(54) **Contrast agent, containing several detection groups per molecule**

(30) Priority: 30.09.1994 JP 261817/94
(71) Applicant: NIHON MEDI-PHYSICS CO., LTD., Hyogo (JP)
(72) Inventor: Ohtaka, Akiharu, Nihon Medi-Physics Co., Ltd., Sodegaura, Chiba, 299-02 (JP); Sugino, Hideki, Nihon Medi-Physics Co., Ltd., Sodegaura, Chiba, 299-02 (JP); Hashiguchi, Yuji, Nihon Medi-Physics Co., Ltd., Sodegaura, Chiba, 299-02 (JP); Seri, Shigemi, Ichihara, Chiba, 299-01 (JP); Iwai, Kumiko, Ichihara, Chiba, 290 (JP)
(74) Representative: von Kreisler, Alek, Dipl.-Chem.

(57) **Abstract**

There is disclosed a diagnostic imaging agent which comprises an oligomer metal complex compound having the molecular weight of 1000 to 10000 and having 2 to 10 functional groups reactive with a bifunctional ligand, wherein at least one kind of and two or more bifunctional ligands are coupled to said organic compound, said organic compound, after coupled to bifunctional ligands, having at least one hydroxyl groups, and said organic compound being coordinated with at least one kind of and two or more metal ions.

## Description

### FIELD OF THE INVENTION

The present invention relates to a nuclear magnetic resonance diagnostic imaging agent and X-ray diagnostic imaging agent, in particular, to an oligomer metal complex compound and the diagnostic imaging agents containing the same.

### BACKGROUND OF THE INVENTION

A nuclear magnetic resonance diagnostic imaging method was first used for the medical image diagnosis in the first half of 1980's. Since then, the nuclear magnetic resonance diagnostic imaging method has made a rapid progress and today it is one of the important diagnostic imaging methods due to their higher spatial resolving ability and a variety of measuring methods.

The nuclear magnetic resonance diagnostic imaging method is to image, as the contrast, the difference in relaxation time of water proton in the tissues. For the purpose of further enhancing this contrast and improving the diagnostic ability, the nuclear magnetic resonance diagnostic imaging agents have been developed. As a nuclear magnetic resonance diagnostic imaging agent, the reagents such as lanthanide ions which have the influence on the relaxation time of water proton have been used. However, since the released lanthanide ions can not be introduced into the body because of the higher toxicity, an attempt was tried to lower the toxicity by complexing them using a physiologically acceptable ligand.

(Diethylenetriaminepentaacetic acid)gadolinate (hereinafter abbreviated as "DTPA-Gd") (JP-A 58-29718) which is currently used most widely as a nuclear magnetic resonance diagnostic imaging agent is obtained by complexing gadolinium ions, which are said to have the largest T1 relaxing effects on water proton among lanthanide ions, with diethylenetriaminepentaacetic acid (hereinafter abbreviated as "DTPA"). This complexation improved the biocompatibility of Gd ions and lowered the toxicity but lowered the relaxivity to the extent of about 1/2 of that of Gd ions themselves. Therefore, there has arisen a need for compensating for this lowered relaxivity with the increased dosage.

As an attempt to improve the efficacy of the relaxivity in order to solve the above problems with regards to DTPA-Gd, the polychelates by the repetition of a monometal complex is described in JP-A 63-41468 and JP-A 2-196776. However, since the polychelates are limited to di-chelates or tri-chelatesn, there still remains the problem on the improvement in the relaxivity.

Thereafter, the use of a polychelate compound obtained by introducing a plurality of metal complexes into a carrier polymer material has been investigated as a diagnostic imaging agent. As the result, a nuclear magnetic resonance diagnostic imaging agent, which uses a polymer having the molecular weight of more than several ten thousand, such as human serum albumin (Ogan M. D. et al.; Invest. Radiol., 22, 665-671 (1987)), dextran (Brasch R. C. et al.: Radiology, 175, 483-488 (1990), JP-A 61-155337, WO-A 8702893), starch (JP-A 61-501571), polylysine (JP-A 64-54028) and the like has been proposed as a prototype imaging agent, which successfully improved the relaxivity.

When a medicine having a polymer as a carrier is designed, the medicine is generally required not to have the immunogenicity and the toxicity as well as retention in the body. However, since it is known that the problems such as antigenicity arise when, for example, polysaccharides having the molecular weight of more than ten thousand are used as a polymer carrier (C. Steffen and H. Ludberg: Clinical Immunology and Allergology, Elsevier Biomedical Press, Amsterdam, 1981, pp. 235-246), the polysaccharides do not comply with the non-immunogenicity requirements in the case of above prototype imaging agent. Further, since all polychelate compounds using the above polymer as a carrier are a polymeric compound having the molecular weight of more than several ten thousand, retention in the blood becomes too longer such as from ten and several hours to several days, which arises a problem. This may not only lead to the bad conditions which help the toxicity expression against antigenicity but also occasionally induce the chemical toxicity resulted from release of metal ions from a ligand by the longer retention and the production of a free ligand. These probabilities should not be overlooked.

### OBJECTS OF THE INVENTION

The main object of the present invention is to solve the above problems observed in a diagnostic imaging agent composed of a polymer polychelate compound, that is, to provide a diagnostic imaging agent, containing an oligomer metal complex compound, which is physiologically acceptable and has the higher imaging ability in comparison with the monochelate compound.

This object as well as other objects and advantages of the present invention will become apparent to those skilled in the art from the following description with reference to the accompanying drawing.

### SUMMARY OF THE INVENTION

The present inventors studied hard and, as the result, we found that an oligomer metal complex compound having a backbone of an organic compound which has at least one hydroxyl group and can be coupled to a bifunctional ligand, wherein 2 to 10 metal complexes are coupled to said backbone organic compound, can solve the above problems. That is, the present invention provides a diagnostic imaging agent which comprises an oligomer metal complex compound having the molecular weight of 1000 to 10000 and having 2 to 10 functional groups reactive with a bifunctional ligand, wherein at least one kind of and two or more bifunctional ligands are coupled to said organic compound, said organic compound, after coupled to bifunctional ligands, having at least one hydroxyl group, and said organic compound being coordinated with at least one kind of and two or more metal ions.

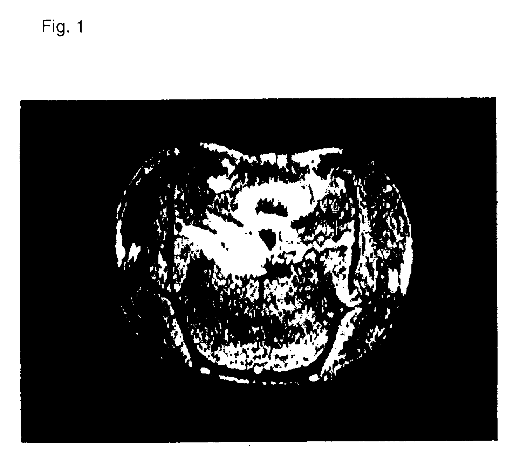

The present oligomer metal complex compound having the molecular weight of 1000 to 10000 can afford better proton relaxation effect in comparison with a monochelate compound. As shown in the below Tables 1 and 2, the oligomer metal complex compound has about 2 to 4 times higher relaxivity when compared with DTPA-Gd. As seen from the imaging effects on a rat brain in the attached Figure 1, the present invention can afford a diagnostic imaging agent, containing an oligomer metal complex, which is useful for imaging diagnosis such as nuclear magnetic resonance imaging diagnosis and the like.

### BRIEF EXPLANATION OF DRAWING

Figure 1 is a MRI showing a transverse view of the head region including the brain of a rat immediately after administration of an OP1-DTPA-Gd solution.

### DETAILED DESCRIPTION OF THE INVENTION

As used herein, the term "oligomer metal complex compound" means a compound where at least 2 and not more than 10 metal complexes are coupled thereto per said molecule.

An organic compound having 2 to 10 functional groups reactive with a bifunctional ligand refers to a straight or branched organic compound, having the molecular weight of not more than 9000, which may contain, in its skeleton, amide linkage, ether linkage, ester linkage, vinyl linkage, acetyl linkage, azo linkage, carbon linkage, imine linkage, imide linkage and the like.

A functional group, contained in the organic compound, reactive with a bifunctional ligand refers to at least one group selected from the group consisting of amino group, carboxyl group, epoxy group, aldehyde group, carbonyl group, thiol group, aryl group, imidazolyl group, hydrazine group, hydroxyl group, halogen atom and the like. Two or more kinds of functional groups may be present.

As a bifunctional ligand, linear or cyclic polyaminopolycarboxylic acids having a crosslinking chain moiety which can couple to the above functional group are used. Preferable examples of the coordinating structure are DTPA and derivatives thereof, 1,4,7,10-tetraazacyclododecane-1,4,7,10-tetraacetic acid and derivatives thereof, 1,4,8,11-tetraazacyclotetradecane-1,4,8,11-tetraacetic acid and derivatives thereof. Preferable examples of a functional group in the crosslinking chain moiety of the bifunctional ligand which may be coupled to the above functional groups are anhydride, amino group, activated halogen, alkoxyester, succinimidiester, isothiocyanate and the like. More particularly, there are anhydrous DTPA, 1,4,7,10-tetraazacyclododecane-1-aminoethylcarbamoylmethyl-4,7,10-tris[(R,S)-methylacetic acid], 1-(p-isothiocyanatobenzyl)-DTPA, 2-(p-isothiocyanatobenzyl)-1,4,7,10-tetraazacyclododecane-1,4,7,10-tetraacetic acid and the like.

Examples of a hydroxyl group contained in the present diagnostic imaging agent may be either a hydroxyl group originally contained in the organic compound having 2 to 10 functional groups reactive with a bifunctional ligand or a hydroxyl group produced by coupling of the organic compound with the bifunctional ligand. Examples of organic compounds containing a hydroxyl group are oligopeptides containing the residues of water-soluble amino acid such as serine (Ser), threonine (Thr), tyrosine (Tyr) and the like (hereinafter amino acid residues are represented using three letters abbreviation), fatty acid derivatives, saccharide derivatives and alcohol derivatives. Examples of a hydroxyl group which is produced by coupling with a bifunctional ligand are a hydroxyl group obtained by reaction of an epoxy polymer with an amino group-containing bifunctional ligand.

The above oligopeptides are commercially available as having the better purity fractionated by chromatography. For example, the desired peptides are prepared by synthesizing them using a peptide synthesizer (manufactured by Applied Biosystem), simultaneously effecting deprotection and cleavage of the peptides bound to the solid phase beads, and thereafter purifying the cleaved peptides with high performance liquid chromatography (HPLC) using a reversedphase column. Alternatively, the desired peptides may be routinely synthesized according to a solid phase peptide synthesis method such as a Fmoc method and the like. By using the fractionated oligopeptides having the high purity, the number of bifunctional ligands and metal ions to be introduced can be definitely controlled. Therefore, pharmaceutically homogenous oligomer metal complex compounds may be prepared.

An organic compound relating to the present invention may be coupled to a bifunctional ligand according to a conventional method. For example, when a crosslinking chain moiety terminal of the bifunctional ligand is an anhydride, an organic compound having an amino group may be stirred with the bifunctional ligand at room temperature in an aqueous alkaline solution to obtain a desired compound. Alternatively, when a crosslinking chain moiety terminal of the bifunctional ligand is an amino group, an organic compound having an epoxy group may be stirred with the bifunctional ligand at room temperature in an aqueous solution to obtain a desired compound. In this case, the coupling reaction between the organic compound and the bifunctional ligand produces a hydroxyl group.

The metal ion in the present invention is selected from the group consisting of metal ions having the atomic number of 21-29, 31, 32, 37-39, 42-44, 49 and 56-83 depending upon a particular use of image diagnosis. When the oligomer metal complex compound of the present invention is used for nuclear magnetic resonance diagnosis, metal ions must be paramagnetic and selected from lanthanide ions having the atomic number of 26 and 57-70, and Gd, Dy, Tb, Ho, Er and Fe being preferable. When the oligomer metal complex compound of the present invention is used for X-ray diagnosis, the metal ion is selected from the group consisting of lanthanide ions having the atomic number of 57-70 and metal ions having the atomic number of 56, 76, 82 and 83. Among them, Bi, Pb and Os ions are preferable. The metal ion to be used may be metal itself or its compound, for example, chloride, oxide and the like. Chelation is carried out according to a conventional method.

The resulting compound contains at least one hydroxyl group, preferably two or more of hydroxyl groups and is composed of an oligomer metal complex compound wherein 2 to 10 bifunctional ligands are chemically coupled thereto and metal ions are coordinated to the ligand moiety. The relaxivity of water proton is remarkably improved in comparison with DTPA-Gd. For example, two DTPA's were bonded to the synthetic peptide, Pyr-Lys-Arg-Pro-Ser-Gln-Arg-Ser-Lys-Tyr-Leu (hereinafter abbreviated as "OP1"), Gd was coordinated therewith to obtain OP1-DTPA-Gd, and its proton relaxivity was determined in vitro. The T1 relaxivity in water was 7.3 (mM·S)⁻¹ and the T2 relaxivity was 8.7 (mM·S)⁻¹. It was confirmed that both relaxivities were remarkably increased to about two times of those of DTPA-Gd. Similarly, two 1,4,7,10-tetraazacyclododecane-1-aminoethylcarbamoylmethyl-4,7,10-tris[(R,S)-methylacetic acid]'s (hereinafter abbreviated as "DO3MA") were coupled to an epoxy polymer (hereinafter abbreviated as "EX-521") represented by the formula 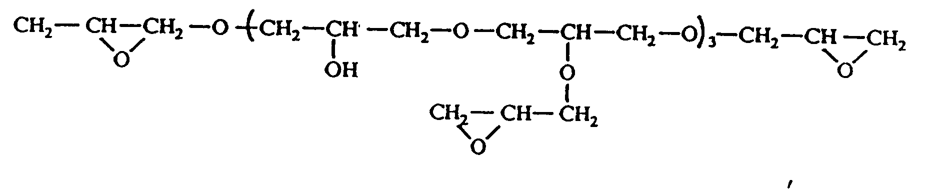
Gd was coordinated therewith to obtain EX-521-DO3MA-Gd, and its relaxivity was determined. In vitro T1 relaxivity was 13.8 (mM·S)⁻¹ and in vitro T2 relaxivity was 17.9 (mM·S)⁻¹. Both relaxivities were remarkably increased to about four times of those of DTPA-Gd.

Such the oligomer metal complex compound may be mixed with an arbitrary additive such as pharmaceutically acceptable stabilizer such as ascorbic acid, p-aminobenzoic acid and the like, pH adjusting agent of aqueous buffer, excipient such as D-mannitol and the like to obtain a diagnostic imaging agent in an arbitrary dosage form. Preferably, the oligomer metal complex compound is dissolved in a physiologically acceptable aqueous solvent to obtain a diagnostic imaging agent in the solution form.

When the oligomer metal complex compound of the present invention is used as a diagnostic imaging agent, the dose to be used is selected depending upon a particular use of image diagnosis. For example, when used as a nuclear magnetic resonance diagnostic imaging agent, the dose of the metal ion is generally 0.0001 to 10 mmol/kg, preferably 0.005 to 0.5 mmol/kg. When used as a X-ray diagnostic imaging agent, the dose of the metal ion is 0.01 to 20 mmol/kg, preferably 0.1 to 10 mmol/kg. Usually, the oligomer metal complex compound is administered intravenously and, in some cases, can be administered orally or intra-arterially.

In addition, since the oligomer metal complex compound of the present invention has good solubility in water due to a hydroxyl group, the compound per se can be prepared into a solution containing the compound in the high concentration. Accordingly, a solubilizer is not necessarily required upon preparation of the solution. The nature of oligomer metal complex compound itself can decrease the total molality in the preparation of a solution in comparison with the mononuclear compound, which results in decrease in osmotic pressure. These alleviate the load to volume of the circulatory system or body fluid equilibrium upon administration in the living body, which results in advantage in the safety.

Following Examples further illustrate the present invention in detail but are not to be construed to limit the scope thereof. The abbreviations used in Examples and analysis method are as follows:
DTPA : diethylenetriaminepentaacetic acid
OP1 : Pyr-Lys-Arg-Pro-Ser-Gln-Arg-Ser-Lys-Tyr-Leu
OP2 : Lys-Thr-Lys-Thr-Lys-Thr-Lys-Thr-Lys
OP3 : Lys-Ser-Lys-Ser-Lys-Ser-Lys-Ser-Lys
DO3MA : 1,4,7,10-tetraazacyclododecane-1-aminoethylcarbamoylmethyl-4,7,10-tris[(R,S)-methylacetic acid]
EX-521: compound represented by the following chemical formula: 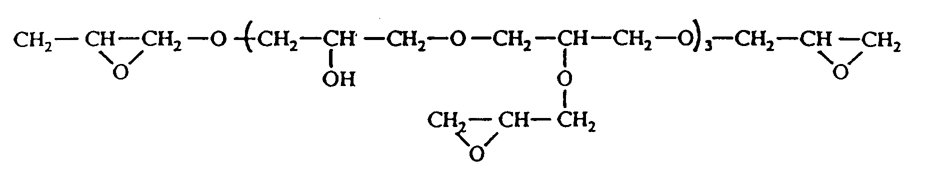
The measurement of ES1 mass spectrum was carried out using a API-III mass spectrometer (manufactured by Perkin Elmer). The measurement of infrared absorption spectrum was carried out according to potassium bromide tablet method using Fourier transform infrared spectrometer FT-IR 8100M (manufactured by Shimazuseisakusho). Relaxation time was measured using JFSE-60 type pulse NMR apparatus (manufactured by Nihondenshi Datum). The concentration of Gd was measured by ICP emission spectrometer SPS-1500VR (manufactured by Seiko).

### Example 1

### Synthesis of OP1-DTPA-Gd

OP1 (41.4 mg; 0.03 mmol) was dissolved in distilled water (4.625 ml) and a 4N aqueous sodium hydroxide solution (0.375 ml) was added thereto. Anhydrous DTPA (0.215 g; 0.6 mmol) was added thereto, followed by reaction while stirring at room temperature for 3 hours. Gadolinium chloride hexahydrate (0.224 g; 0.6 mmol) was added to the reaction solution and a 4N aqueous sodium hydroxide solution was added thereto at an appropriate amount to adjust pH to about 7. The reaction solution was reacted while stirring at room temperature for 15 minutes, followed by purification by electrodialysis to obtain OP1-DTPA-Gd (55.4 mg). The results of analysis are as follows:
ES1-mass spectrum (positive ion): m/z 812 (trivalent ion), m/z 1217 (divalent ion)
Infrared absorption spectrum: 1700 cm⁻¹ (CO), 3000 cm⁻¹ (CH), 3200 cm⁻¹ (NH)

### Example 2

### Synthesis of OP2-DTPA-Gd

OP2 (95 mg; 0.089 mmol) was dissolved in distilled water (3.89 ml) and a 4N aqueous sodium hydroxide solution (1.11 ml) was added thereto. Anhydrous DTPA (0.636 g; 1.78 mmol) was added thereto, followed by reaction while stirring at room temperature for 3 hours. Gadolinium chloride hexahydrate (0.662 g; 1.78 mmol) was added to the reaction solution, and a 4N sodium hydroxide solution was added thereto at an appropriate amount to adjust pH to about 7. The reaction solution was reacted while stirring at room temperature for 15 minutes, followed by purification by electrodialysis to obtain OP2-DTPA-Gd (48.2 mg). The results of analysis are as follows:
ESI-mass spectrum (positive ion): m/z 1050 (trivalent ion), m/z 1580 (divalent ion)
Infrared absorption spectrum: 1700 cm⁻¹ (CO), 3000 cm⁻¹ (CH), 3200 cm¹ (NH)

### Example 3

### Synthesis of OP3-DTPA-Gd

OP3 (45 mg; 0.045 mmol) was dissolved in distilled water (4.44 ml) and a 4N aqueous sodium hydroxide solution (0.56 ml) was added thereto. Anhydrous DTPA (0.319 g; 0.9 mmol) was added thereto, followed by reaction while stirring at room temperature for 3 hours. Gadolinium chloride hexahydrate (0.332 g; 0.9 mmol) was added to the reaction solution, and a 4N aqueous sodium hydroxide solution was added thereto at an appropriate amount to adjust pH to about 7. The reaction solution was reacted while stirring at room temperature for 15 minutes, followed by purification by electrodialysis to obtain OP3-DTPA-Gd (48.3 mg). The results of analysis are as follows:
Infrared absorption spectrum: 1700 cm⁻¹ (CO), 3000 cm⁻¹ (CH), 3200 cm⁻¹ (NH)

### Example 4

### Synthesis of EX-521-DO3MA-Gd

EX-521 (0.1 g; 13.5 mmol) was dissolved in distilled water (1.0 ml) and DO3MA-Gd (0.5 g; 77.9 mmol) was added thereto to stir at room temperature. After 26 hours, EX-521 (0.05 g; 13.5 mmol) was further added thereto to further stir for 70 hours. Thereafter, the reaction solution was dialyzed for 48 hours using a dialysis membrane having fractionation molecular weight of 1,000 to remove unreacted DO3MA-Gd. The solution in the dialysis membrane was concentrated, and dried under reduced pressure to obtain the desired crystals (0.34 g) (yield: 50.7%). The results of analysis are as follows:
Infrared absorption spectrum: 1600 cm⁻¹ (COO-), 2830 cm⁻¹ (CH₂), 3250 cm⁻¹ (OH)
Gd concentration (ICP emission analysis): 12.5 wt%

### Example 5

### Relaxivity (in vitro experiment)

OP1-DTPA-Gd, OP2-DTPA-Gd, EX-521-DO3MA-Gd and DTPA-Gd (control) were dissolved in distilled water to adjust the Gd concentration to 4 mM, respectively. The relation with water proton exposed to the compounds was determined as relaxation time at 37 °C (T1 and T2 msec) using a NMR apparatus (manufactured by Nihondenshi Datum, 0.5T). Each relaxation time and relaxivity (R1 (mM·S)⁻¹, R2 (mM·S)⁻¹ calculated therefrom are shown in Tables 1 and 2.

The oligomer metal complex compounds of the present invention have the remarkable relaxation time shortening effects as follows: 3.8 mM OP1-DTPA-Gd has about 90 times T1 value of that of water and about 73 times T2 value of that of water, 4.0 mM OP2-DTFA-Gd has about 112 times T1 value of that of water and about 110 times T2 value of that of water, and 4.0 mM EX-521-DO3MA-Gd has about 182 times T1 value of that of water and about 159 times T2 value of that of water.

In addition, OP1-DTPA-Gd, OP2-DTPA-Gd and EX-521-DO3MA-Gd have better, about 2 to 4 times relaxivity of that of DTPA-Gd measured according to the same in vitro method, respectively. Thus, the efficacy of oligomer metal complex compounds of the present invention was confirmed.

**Table 1**

| Relaxation time | | | |
|---|---|---|---|
| Compound | Concentration (mM) | T1 (msec) | T2 (msec) |
| OP1-DTPA-Gd | 3.8 | 36.2 | 30.3 |
| OP2-DTPA-Gd | 4.0 | 29.2 | 20.0 |
| EX-521-DO3MA-Gd | 4.0 | 18.0 | 13.9 |
| DTPA-Gd | 4.0 | 15.1 | 18.5 |
| Control (Water) | 0 | 3275 | 2208 |

**Table 2**

| Relaxivity | | |
|---|---|---|
| Compound | R1 (mM·S)⁻¹ | R2 (mM·S)⁻¹ |
| OP1-DTPA-Gd | 7.3 | 8.7 |
| OP2-DTPA-Gd | 8.5 | 12.4 |
| EX-521-DO3MA-Gd | 13.8 | 17.9 |
| DTPA-Gd | 3.7 | 4.5 |

### Example 6

### The imaging effect of OP1-DTPA-Gd on the rat brain after intravenous administration

An ethylnitrosourea-induced brain tumored rat (230.5 g, 42 weeks old) anesthetized with thiopental was fixed prone in the magnetic field of a MRI apparatus and an OP1-DTPA-Gd solution (Gd concentration: 0.2 M) was administered via tail vein (0.1 mmol Gd/kg). Immediately after administration, the MRI measurement (transverse) of the head region including the brain was conducted.

The apparatus (Omega CSI manufactured by Bruker) had magnetic field intensity of 2.0T and, as an imaging coil, a bird-cage type head high-pass coil was used. Imaging was carried out according to spin echo method of T1 weighted (TR=600 msec, TE=12 msec) under the condition of 2 mm in slice thickness, a resolution of 256 x 128.

Figure 1 is a MRI showing a transverse view of the head region including the brain of a rat immediately after administration of an OP1-DTPA-Gd solution. It is clearly understood that administration potentiated the signal intensity from the brain tumor part.

## Claims

1. A diagnostic imaging agent which comprises an oligomer metal complex compound having the molecular weight of 1000 to 10000 and having 2 to 10 functional groups reactive with a bifunctional ligand, wherein at least one kind of and two or more bifunctional ligands are coupled to said organic compound, said organic compound, after coupled to bifunctional ligands, having at least one hydroxyl group, and said organic compound being coordinated with at least one kind of and two or more metal ions.

2. A diagnostic imaging agent according to claim 1, wherein the functional group reactive with the bifunctional ligand is at least one group selected from the group consisting of amino group, carboxyl group, epoxy group, aldehyde group, carbonyl group, thiol group, aryl group, imidazolyl group, hydrazine group and halogen atom.

3. A diagnostic imaging agent according to claim 1 or 2, wherein the bifunctional ligand is diethylenetriaminepentaacetic acid, 1,4,7,10-tetraazacyclododecane-1,4,7,10-tetraacetic acid, or 1,4,8,11-tetraazacyclotetradecane-1,4,8,11-tetraacetic acid or derivatives thereof.

4. A diagnostic imaging agent according to any one of claims 1 to 3, wherein the metal ion is selected from the atomic number 26 and the group consisting of lanthanide ions having the atomic number of 57-71.

5. A diagnostic imaging agent according to claim 4, wherein the metal ion is Gd, Dy, Ho, Er or Fe ion.

6. A diagnostic imaging agent according to any one of claims 1 to 3, wherein the metal ion is Bi, Pb or Os ion.
